# FASCICULE DE BREVET EUROPEEN

(11) **EP 0 988 085 B1**
(45) Date de publication et mention de la délivrance du brevet: **13.10.2004**
(21) Numéro de dépôt: 97929352.9
(22) Date de dépôt: 13.06.1997
(51) Int. Cl.: A61N 1/16, A61N 1/40

(54) **DISPOSITIF ELECTROMAGNETIQUE DE STIMULATION CUTANEE POUR LE TRAITEMENT D'ETATS PATHOLOGIQUES**
VORRICHTUNG ZUR BEHANDLUNG VON PATHOLOGISCHEN ZUSTÄNDEN MITTELS ELEKTROMAGNETISCHER HAUTSTIMULATION
DEVICE FOR ELECTROMAGNETIC CUTANEOUS STIMULATION FOR TREATING PATHOLOGICAL CONDITIONS

(43) Date de publication de la demande: 29.03.2000
(73) Titulaire: Baudry, Alain, 13009 Marseille (FR); MARIGNAN, Michel, F-13011 Marseille (FR)
(72) Inventeur: Baudry, Alain, 13009 Marseille (FR); MARIGNAN, Michel, F-13011 Marseille (FR)
(74) Mandataire: Domange, Maxime
(86) Numéro de dépôt international: PCT/FR1997/001074
(87) Numéro de publication internationale: WO 1998/056457

(56) Documents cités:
- EP-A- 0 065 473
- WO-A-95/03850
- FR-A- 2 620 943
- FR-A- 2 625 105
- JUNGE: "Lexikon Elektronik", 15. Mars 1978, PHYSIK VERLAG, LEIPZIG

## Description

La présente invention concerne un nouveau dispositif électromagnétique de stimulation cutanée visant à modifier un état physiologique ou réaliser le traitement d'une pathologie donnée, ainsi qu'un dispositif électronique permettant de mettre en oeuvre ce procédé.

Les techniques de traitement de diverses pathologies, par stimulation externe de points ou de zones spécifiques du corps sont nombreuses. Les stimulations connues aujourd'hui sont réalisées par des dispositifs utilisant diverses techniques, parmi lesquelles on pourra citer les aiguilles utilisées traditionnellement en acupuncture, les vibrations mécaniques, les courants électriques, le rayonnement électromagnétique, les aimants, le rayonnement lumineux (lumière visible non cohérente, laser, ultraviolets et infrarouges..), les filtres optiques, etc.

Les points ou surfaces de stimulation utilisés sont nombreux, répartis sur quasiment la totalité de la surface corporelle, et l'on peut citer sans que cela soit limitatif, les points connus en acupuncture, en réflexo-thérapie, auriculo-médecine et dans les dispositifs podologiques.

Pour illustrer la diversité et l'état de ces techniques, on peut citer quelques brevets représentatifs dans ce domaine :
1 - Le brevet Français déposé le 10 Février 1975, sous le N° 75 04116 par la société ELMATRON G.M.B.H. (RFA), concernant un "appareil pour favoriser la guérison des cellules d'organisme humains et animaux au moyen d'impulsions électromagnétiques" Il s'agit la d'un appareil qui émet des ondes électromagnétiques de fréquence non précisée (hautes fréquences), mais sous forme d'impulsions répétitives à des fréquences de 0 à 1000 Hz.
2 - Le brevet allemand du 29 Octobre 1975, étendu à la France le 25 octobre 1976 sous le N° 76/32.116 par la Société MESSERSCHMITT-BOLKOW-BLOHM concernant un "appareil faisant appel à l'action de la lumière pour des traitements thérapeutiques semblables à l'acupuncture". Il s'agit en fait d'une projection de rayonnement laser de faible puissance pulsé par un obturateur suivant une fréquence de 2 à 20 Hz, stimulant des points précis (point lumineux de l'ordre de 1mm de diamètre), déterminés en acupuncture et donnant les mêmes effets qu'avec des aiguilles.
3 - Le brevet français déposé le 24 Novembre 1976 sous le N° 76/36 152 par Mr. Pierre NOGIER concernant un "procédé et appareil pour stimulation locale par rayonnement électromagnétique". Ce rayonnement est en fait produit par un faisceau au moins semi-cohérent, de rayons infrarouges, dont la fréquence de base est à 73 Hz, et comportant de nombreuses harmoniques, et éclairant des points choisis du corps non précisés et sans donner de précision sur les effets obtenus.
4 - Le brevet français déposé le 15 Février 1979 sous le N° 79/04.486 par Mr. P. NOGIER décrivant un "procédé et appareil pour le traitement magnétique d'organismes vivants". Il s'agit d'un dispositif propre à polariser la lumière, de type verre Polaroïd, interposé entre une source de flux magnétique de type aimant et l'organisme à traiter; le flux polarisé pénètre alors profondément dans l'organisme sans perdre sa polarisation contrairement à la lumière polarisée. Les appareils utilisant ce procédé sont commercialisés sous le nom de "POLARTRON" et agissent en fait sur le système nerveux pour calmer les douleurs (oreilles, cicatrices etc...).
5 - Le brevet français déposé le 28 Octobre 1980 sous le N° 80/23.007 par Mr. Christian C. MARET concernant un "appareil à émission lumineuse ayant un effet physiologique par voie cutanée". Cet appareil est assez complexe, car il comprend un émetteur de lumière clignotante suivant une période de 2 à 6 secondes, traversant successivement et dans cet ordre un ensemble de filtres colorés, puis de couches de tissu imprégné de substances actives diluées.
   Ce dispositif est plutôt fixé au poignet ou à la cheville de l'utilisateur et permet de combiner plusieurs actions thérapeutiques par un choix approprié des filtres associés aux substances actives diluées et dynamisées selon les méthodes d'HAHNEMANN.
6 - Le brevet français déposé le 13 mai 1981 sous le N° 81/09.663 et étendu à l'Europe sous le N° 82430023.1 par Mr. Bernard BRICOT décrivant un "dispositif podal pour le traitement des zones réflexes des pieds et notamment des troubles et affections dus au déséquilibre rachidien". Il s'agit ici de stimuler des zones réflexes des pieds, assez générales du reste puisque le dispositif revendiqué peut couvrir toute la surface plantaire, par un polariseur constitué par au moins deux plaques ou feuilles d'une matière ayant une orientation moléculaire ou cristalline déterminée, ces plaques étant superposées de telle façon que leur axe de polarisation soit croisé. Ce dispositif utilise en fait le même principe thérapeutique que le POLARTRON décrit dans le brevet de Mr. NOGIER, mais sans source active magnétique qui est toxique si l'intensité est trop forte, surtout en utilisation podale. Les résultats thérapeutiques sont obtenus au bout de quelques mois par un rééquilibrage du rachis et des améliorations de quelques perturbations fonctionnelles en rapport avec la statique.
7 - Le brevet allemand déposé le 22 Juillet 1983 sous le N° 33 26 513.5 et étendu à l'Europe dont la France, par Mr. TETZNER Volkmar, concernant un "appareil d'irradiation pour des traitements photobiologiques et photochimiques". Cet appareil comprend une source d'éclairage ultraviolet éclairant la peau pour des traitements en particulier dermatologiques, à travers un jeu de filtres placés dans un boîtier, deux à deux, pouvant être interchangés facilement suivant le traitement.
8.- Le brevet français déposé le 29 Août 1983 sous le N° 83 14020 par Mr. MOREZ Jean Bernard, concernant un "appareil pour la stimulation non manuelle de points du revêtement: cutané appelés points d'acupuncture". Cette invention concerne une stimulation acoustique modulée en basse fréquence.
9 - Le brevet français déposé le 17 juin 1988 sous le N° 88 08347 par Mrs. MARIGNAN Michel, REBOUL Philippe et SOUVESTRE Philippe, concernant un "dispositif podologique pour la correction des troubles et des affections du tonus neuromusculaire de posture". Ce dispositif est constitué d'un ou plusieurs films agissant comme filtres optiques et placés dans une semelle le positionnant en regard de la zone réflexe que l'on désire stimuler.
10 - Le brevet Français déposé le 18 mai 1990 sous le N° 90 06241, puis étendu à l'international le 14 Mai 1991 sous le N° WO 91/17737 par Mr. VU-DINH Sao, concernant un "Appareil portable autonome d'acupuncture". Cet appareil de petite dimension intégré dans un boîtier de type montre bracelet, réalise sur des points de la face interne du poignet, une stimulation électrique, par l'application de tensions de 1 à 12 volts, d'une durée de 0,1 à 2 secondes, avec une fréquence de répétition de 0,5 à 10 Hz.
11 - Le brevet Européen déposé le 2 Octobre 1990 sous le N° 0 447 568 A1 par Mrs. LOBAREV Valery, SITKO Sergei, LJUCHENKO Vadim et décrivant un "appareil pour la thérapie par résonance de Micro-ondes. Cet appareil est constitué d'une source électromagnétique dans la bande millimétrique (25 à 150 GHz) qui provoquerait une stimulation des fréquences de résonance de l'organisme.

La plupart de ces dispositifs ou procédés utilisent une source active externe, ce qui en complique l'utilisation (encombrement, poids) et en interdit le port permanent, car il faut une source d'énergie. De plus, leur action peut être toxique car l'intensité du rayonnement peut avoir un effet négatif sur l'organisme.

La stimulation est quelquefois continue, mais aussi souvent pulsée ou modulée par un signal basse fréquence.

Aucun de ces équipements n'a du reste des résultats statistiquement probants et réguliers.

Plusieurs de ces dispositifs utilisent des émissions électromagnétiques, notamment dans le spectre de la lumière visible ou infrarouge.

Les dispositifs de ce type sont parfois complétés par l'interposition de filtres (polarisant ou coloré) qui permettent d'optimiser les effets positifs de la dite source, dont certaines sont utilisées directement sur l'organisme (laser, lampe ultraviolet, champ magnétique etc....).

Ces filtres sont rajoutés pour limiter en fait le rayonnement de la source en ne laissant passer que la partie active du flux de celui-ci, qui est alors renforcé.

Par ailleurs, l'application de ces appareils se fait en des points qui ne sont jamais bien précisés par les inventeurs, qui ont en général constaté des effets bénéfiques sur les organismes, mais sans résultats ni tests prouvés et décrits dans leur invention, car effectivement, ceux-ci peuvent être très différents d'une personne à l'autre et c'est l'expérience de l'utilisateur qui montre à ceux-ci les zones du corps les plus réceptives et les traitements correspondants.

Seules quelques techniques n'utilisent pas de source active externe, tels l'invention de Mr. BRICOT qui utilise des plaques polarisantes, celle de Mrs. MARIGNAN et SOUVESTRE qui utilise des filtres optiques, ou celles qui utilisent des aimants comme l'invention de Mr. LEBART Edouard qui combine aimants et stimulation mécanique (Brevet Français 2 687 075 du 13 08 1993).

Le document Lexikon Elektronic, Physic Verlay 1978, page 5 décrit un circuit électrique résonnant.

La présente invention a pour objet un dispositif tel que défini dans les revendications 1 à 8.

L'objet de la présente invention est un dispositif electromagnétique de stimulation cutanée, appliqué à un organisme vivant et plus particulièrement l'organisme humain, en certains points ou certaines surfaces déterminés par la pathologie à traiter, ou les modifications physiologiques recherchées, utilisant un dispositif électronique résonant, dont la ou les fréquences de résonance (appelées aussi fréquences propres) seront elles mêmes déterminées par l'effet recherché et attendu de la stimulation.

Par opposition aux procédés connus de stimulation par génération de champ magnétique ou émission d'ondes électromagnétiques à l'aide d'une source active, qui transfère de l'énergie à l'organisme, l'invention permet de réaliser un procédé de stimulation dans lequel on utilise la capacité de résonance d'un dispositif passif pour interagir avec l'organisme, principalement en absorbant de l'énergie électromagnétique externe au dispositif.

Le procédé consiste donc à placer un dispositif électronique en certains endroits propices à une stimulation, de telle sorte que le dit dispositif influencé par le champ magnétique et le rayonnement électromagnétique engendré par l'organisme, entre en résonance à sa ou ses fréquences propres, laquelle ou lesquelles étant prédéterminées suivant l'effet recherché sur cet organisme et le dispositif réalisé en conséquence.

Le procédé met en jeu un couplage électromagnétique entre le dispositif selon l'invention, et toutes les structures de l'organisme susceptibles d'interagir avec le dispositif au travers de ce couplage.

La résonance du circuit induite par l'organisme engendre une modification du comportement électromagnétique de l'organisme, et provoque une stimulation de cet organisme.

Le procédé ne nécessite donc pas de contact direct avec l'organisme; il peut être mis en oeuvre par un dispositif éventuellement totalement passif c'est à dire sans source d'énergie, et donc sans risque de dépasser les énergies admissibles pour l'organisme.

L'intérêt d'un tel procédé est de permettre une stimulation permanente, efficace mais inoffensive, qui peut être appliquée en tout les points du corps y compris la plante des pieds, au moyen de dispositifs dont la taille très réduite permet un port continu et discret.

Par ailleurs la fréquence de résonance voulue, liée à la pathologie traitée, peut être aisément obtenue dans un dispositif selon l'invention, de même que toutes les harmoniques de cette fréquence.

Ce procédé de stimulation, par son action efficace et prolongée a démontré son efficacité d'abord palliative, puis à plus long terme curative dans différentes pathologies.

On peut citer à titre d'exemple des résultats contrôlés et mesurés, spectaculaires, obtenus sur des individus souffrant de troubles de l'équilibre, consécutifs à des atteintes graves de l'oreille interne comme par exemple des fistules péri-lymphatiques.

Une autre application ayant montré l'efficacité des dispositifs est la correction des troubles et affections du tonus neuromusculaire de posture.

Le dispositif objet de cette invention, permettant la mise en oeuvre du procédé décrit ci-dessus peut être réalisé par un ou plusieurs circuits électroniques ayant un comportement résonant, et un élément de couplage électromagnétique avec les structures concernées de l'organisme.

Un tel circuit présente un mode de fonctionnement dans lequel lorsqu'il est en résonance, il absorbe de l'énergie à l'organisme qui se comporte alors comme une source, cette énergie étant amplifiée par le fait que cet échange s'effectue à certaines fréquences particulières au circuit et dites fréquences de résonances. Ce phénomène est bien connu et utilisé en mécanique et en électronique.

Le couplage électromagnétique est réalisé par induction au moyen d'un élément de type bobinage, ou à l'aide d'un dispositif de type dipôle.

De tels dispositifs comprennent des composants électroniques actifs ou passifs. On peut aisément réaliser de tels circuits avec des composants totalement passifs : Capacité, self-inductance, résistance.

Le choix des composants et de leur valeur est effectué en fonction des objectifs biologiques et médicaux à atteindre; il porte sur la ou les fréquences de résonance, ainsi que sur l'intensité du couplage.

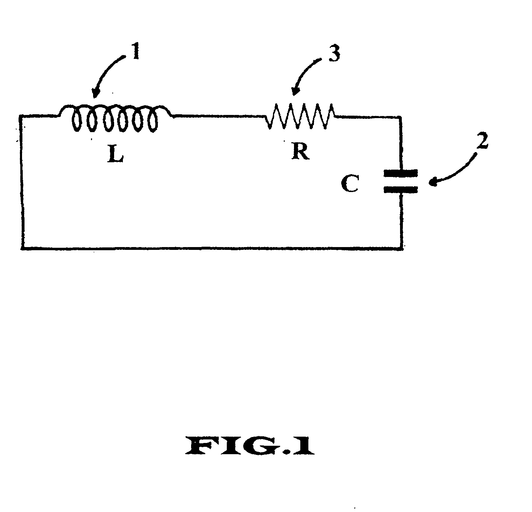

Un des dispositifs les plus simples permettant de réaliser le procédé décrit, comporte en circuit fermé, une self-inductance L, **1**, pouvant être réalisée par quelques spires d'un bobinage, une capacité C, **2**, et une résistance R, **3** (fig. 1)

Pour obtenir un couplage plus efficace, la self inductance sera placée de telle sorte que le plan contenant les spires soit parallèle au plan tangent à la surface de la peau, au point que l'on désire stimuler.

Un tel circuit possède une fréquence propre d'oscillation F, définie par : F = 1 / [2π(LC)^{1/2}]

Un procédé avantageux pour réaliser la self-inductance, composant intervenant dans la résonance et dans le couplage magnétique avec l'organisme, consiste à réaliser un circuit imprimé sur un support plan 4, qui peut être rigide ou souple, dans lequel une piste du circuit par un trajet concentrique, réalise les spires circulaires ou polygonales emboîtées de cette self-inductance.

Dans ce cas de réalisation, le circuit sera placé de telle sorte que la plaque du circuit imprimé, soit paralléle à la surface cutanée, comme indiqué précédemment.

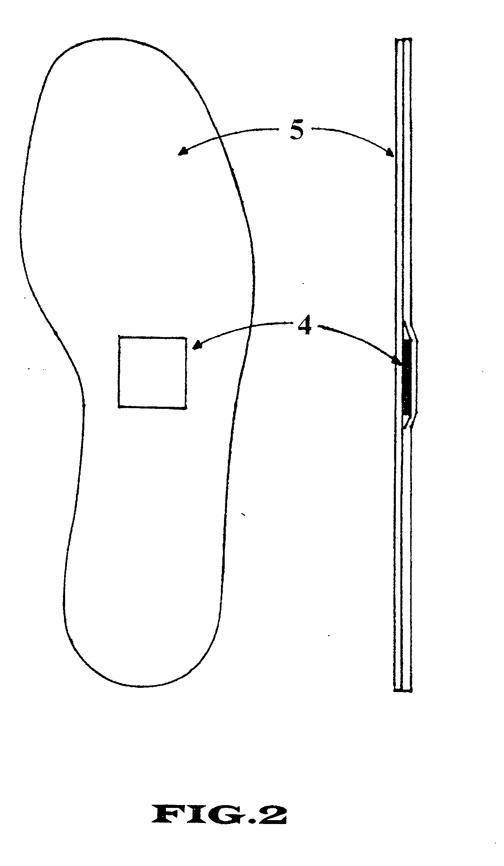

Ce mode de réalisation et de mise en place du dispositif peut être illustré par un circuit LRC comme indiqué précédemment, dont une piste circulaire réalise les spires concentriques, situées dans le plan du circuit imprimé, et qui implanté dans une semelle 5, (fig 2.) à placer sous la plante du pied permet un couplage optimal.

Un exemple de réalisation du dispositif comme décrit ci dessus est obtenu par un circuit LRC dont la self inductance réalisée au moyen d'une piste en cuivre du circuit imprimé formant les spires a une valeur de l'ordre de 0,5 µH associée à un condensateur de 12 nF qui présente donc une fréquence de résonance de l'ordre de 2MHz.

Un tel dispositif exemple d'application non limitatif de la présente invention a montré son efficacité pour le traitement des vertiges et malaises associés dans des cas de fistules périlymphatiques. D'autres fréquences sont également utilisées pour d'autres pathologies, principalement mais sans être limitatif dans le domaine des Fréquences de quelques MégaHerz à quelques dizaines de MégaHerz.

Lorsque la résistance R du circuit est très faible, et par exemple constituée uniquement de la résistance propre des pistes du circuit imprimé support du dispositif, la résonance se situe dans une bande de fréquence très étroite, elle est de forte amplitude, et permet une stimulation très sélective en fréquence et très puissante.

Par contre une résistance non négligeable dans le circuit est un facteur d'amortissement de la résonance qui permet de stimuler de façon plus douce.

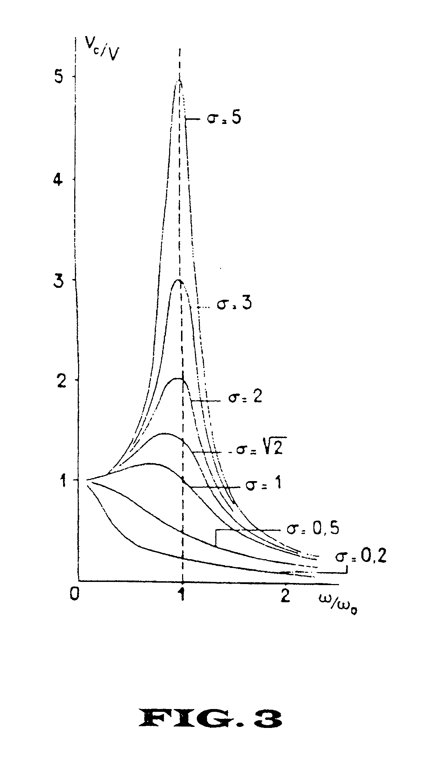

Le schéma (fig. 3) montre le phénomène de résonance pour différentes valeurs du terme d'amortissement lié à cette résistance.

On peut y voir l'importance du facteur d'amplification V_{c}/V en ordonnée (rapport de la tension V_{c} dans le circuit à la force électromotrice appliquée ou induite V) en fonction du rapport des pulsations ω/ω₀ en abscisse (rapport de la pulsation ω de la force électromotrice appliquée à la fréquence propre du circuit ω₀ = 2πF ) pour différentes valeurs du coefficient d'amortissement σ = Lω₀ / R.

Des dispositifs selon l'invention, plus complexes, peuvent être réalisés en associant plusieurs circuits du type précédent (monomode, c'est à dire avec une seule fréquence de résonance), ou en utilisant des circuits multimodes, ce qui permet de stimuler l'organisme à plusieurs fréquences simultanément.

Des fonctions de stimulation plus complexes peuvent enfin être réalisées en associant des composants actifs dans un circuit résonant.

On peut de même prévoir d'associer au dispositif des fonctions supérieures de type auto-test, et des caractéristiques évolutives et modulables dans le temps.

Compte tenu de la diversité des points ou zones de l'organisme qui peuvent être soumis à une stimulation selon le procédé décrit, le dispositif qui peut être de dimension très réduite compte tenu de son principe, sera inséré dans un support qui permet de le maintenir en place à faible distance du point à stimuler, mais sans qu'un contact physique soit nécessaire.

Les tests réalisés on été effectués au moyen des supports indiqués ci-après, sans que cela soit limitatif :
- semelle pour une stimulation podale (fig.2).
- bracelet pour une stimulation au poignet.
- bandeau pour une stimulation crânienne.
- bandage ou ruban adhésif pour d'autres localisations.

Cette invention s'applique bien évidemment, mais non limitativement, au domaine de la médecine humaine et vétérinaire.

## Revendications

1. Dispositif pour la stimulation cutanée en certains points ou zones de l'organisme, comprenant un dispositif électromagnétique incorporé dans un support permettant de placer ledit dispositif électromagnétique à proximité desdits points ou de ladite zone à stimuler, **caractérisé en ce que** ledit support est constitué par une semelle, un bracelet, un bandeau, un bandage ou un ruban adhésif et ledit dispositif électromagnétique est un dispositif électronique constitué d'un circuit passif résonant suivant au moins une fréquence propre de résonance, dont ladite résonance est induite par les rayonnements électromagnétiques de l'organisme, lequel circuit ne comporte pas de source d'énergie et comporte un élément de couplage électromagnétique avec lesdits points ou ladite zone de l'organisme que l'on désire stimuler.

2. Dispositif selon la revendication 1, **caractérisé en ce que** ledit élément de couplage électromagnétique avec l'organisme est de type bobine à induction ou de type antenne.

3. Dispositif selon la revendication 1 ou 2, **caractérisé en ce que** ledit circuit électronique comporte une bobine d'induction (1), une capacité (2) et une résistance (3), lesdits composants étant disposés soudés sur un circuit imprimé plan rigide ou souple (4), et ladite bobine étant constituée par une piste dudit circuit imprimé dont le trajet forme un ensemble de spires concentriques.

4. Dispositif selon la revendication 3, **caractérisé en ce que** ledit circuit et ladite bobine sont placés de telle sorte que le plan contenant les spires de ladite bobine soit parallèle au plan tangent à la surface de la peau au point que l'on désire stimuler, ladite bobine faisant alors office d'élément de couplage électromagnétique avec l'organisme.

5. Dispositif selon l'une des revendications précédentes, **caractérisé en ce que** ladite fréquence propre de résonance est choisie en fonction d'une pathologie à traiter ou d'une situation physiologique à modifier donnée.

6. Dispositif selon la revendication 1, **caractérisé en ce que** ledit support est une semelle.

7. Dispositif selon l'une des revendications 3 et suivantes, **caractérisé en ce que** ladite bobine est réalisée au moyen d'une piste en cuivre du circuit imprimé formant les spires, et à une valeur de l'ordre de 0,5 µH associé à un condensateur de 12 nF qui présente donc une fréquence de résonance de l'ordre de 2 MHz.

## Claims

1. A device for the stimulation of the skin at various points or zones of the organism, comprising an electromagnetic device incorporated in a support for positioning said electromagnetic device close to said points or zone to be stimulated, **characterised in that** said support consists of a sole, bracelet, band, bandage or adhesive tape and said electromagnetic device is an electronic device constituted by a passive resonant circuit which has at least one proper resonant frequency, the resonance of which is being induced by the electromagnetic radiation of the organism, which circuit does not have a source of energy and comprises an electromagnetic coupling element with said point or zone of the organism that is to be stimulated.

2. A device according to claim 1, **characterised in that** said electromagnetic coupling element with the organism is of the induction coil type or of the antenna type.

3. A device according to claim 1 or 2, **characterized in that** said electronic circuit comprises a self-inductor (1), a capacitor (2), and a resistor (3), said components being disposed and soldered on a plane printed circuit (4) that is rigid or flexible and said coil being constituted by a track of said printed circuit whose path forms a set of nested turns.

4. A device according to claim 3, **characterised in that** said circuit and said coil are placed in such a way that the plane containing the turns of said coil is parallel to the tangent plane at the surface of the skin at the point that is desired to be stimulated, said coil then acting as an electromagnetic coupling element with the organism.

5. A device according to one of the preceding claims, **characterised in that** said proper resonant frequency is selected as a function of a pathology to be treated or of a given physiological condition to be changed.

6. A device according to claim 1, **characterised in that** said support is a sole.

7. A device according to one of claims 3 et seq., **characterised in that** said coil is made by means of a copper track of the printed circuit forming the turns, and at a value of about 0.5 µH combined with a condenser of 12 nF which thus has a resonant frequency of about 2 MHz.

## Patentansprüche

1. Vorrichtung für die Hautstimulation an bestimmten Punkten oder Bereichen des Organismus mit einer elektromagnetischen Vorrichtung, die in einen Träger eingebaut ist, der diese elektromagnetische Vorrichtung in der Nähe der zu stimulierenden Punkte bzw. Bereiche zu plazieren erlaubt,
**dadurch gekennzeichnet, dass** der Träger aus einer Sohle, einem Armband, einem Stirnband, einer Bandage oder einem Klebeband gebildet ist, und die elektromagnetische Vorrichtung eine elektronische Vorrichtung ist, die aus einem passiven Schwingkreis gemäß wenigstens einer Eigenresonanzfrequenz gebildet ist, deren Resonanz durch die elektromagnetischen Strahlungen des Organismus induziert wird, wobei der Schwingkreis keine Energiequelle aufweist und ein Element zur elektromagnetischen Kopplung mit den Punkten bzw. Bereichen des Organismus umfasst, die stimuliert werden sollen.

2. Vorrichtung nach Anspruch 1,
**dadurch gekennzeichnet dass** das Element zur elektromagnetischen Kopplung mit dem Organismus eine Induktionsspule oder eine Anntenne ist

3. Vorrichtung nach Anspruch 1 oder 2,
**dadurch gekennzeichnet, dass** die elektronische Schaltung eine Induktionsspule (1), eine Kapazität (2) und einen Widerstand (3) umfasst, wobei diese Bauteile aufgeschweißt auf eine ebene starre oder biegsame gedruckte Schaltung (4) angeordnet sind und wobei die Spule durch eine Spur der gedruckten Schaltung gebildet ist, deren Verlauf eine Gesamtheit von konzentrischen Windungen bildet.

4. Vorrichtung nach Anspruch 3,
**dadurch gekennzeichnet, dass** die Schaltung und die Spule so angeordnet sind, dass die Ebene, die die Windungen der Spule enthält, parallel zu der Ebene ist, die die Oberfläche der Haut in dem Punkt berührt, der stimuliert werden soll, wobei die Spule somit als Element zur elektromagnetischen Kopplung mit dem Organismus dient.

5. Vorrichtung nach einem der vorhergehenden Ansprüche,
**dadurch gekennzeichnet, dass** die Eigenfrequenzresonanz in Abhänglgkeit von einer zu behandelnden Pathologie oder einem gegebenen zu ändernden physiologischen Zustand gewählt ist.

6. Vorrichtung nach Anspruch 1,
**dadurch gekennzeichnet**, das der Träger eine Sohle ist.

7. Vorrichtung nach einem der Ansprüche 3 und folgende,
**dadurch gekennzeichnet, dass** die Spule mittels einer die Windungen bildenden Kupferspur der gedruckten Schaltung mit einem Wert von etwa 0,5 µH ausgeführt ist und einem Kondensator von 12 nF zugehörig ist, der somit eine Resonanzfrequenz von etwa 2 MHz aufweist.
